# FASCICULE DE BREVET EUROPEEN

(11) **EP 3 344 224 B1**
(45) Date de publication et mention de la délivrance du brevet: **31.07.2019**
(21) Numéro de dépôt: 16760443.8
(22) Date de dépôt: 30.08.2016
(51) Int. Cl.: A61K 8/34, A61K 8/36, A61K 8/365, A61Q 3/02, A61K 8/85

(54) **COMPOSITION COSMÉTIQUE ANHYDRE**
ANHYDRISCHE KOSMETISCHE ZUSAMMENSETZUNG
ANHYDROUS COSMETIC COMPOSITION

(30) Priorité: 02.09.2015 LU 92811
(43) Date de publication de la demande: 11.07.2018
(73) Titulaire: International Lacquers S.A., 3225 Bettembourg (LU)
(72) Inventeur: ROVILLE, Charlène, SW11 2NW London (GB); DEROSIER, Frédéric, 57680 Corny sur Moselle (FR); DELAS, Christophe, 3926 Mondercange (LU)
(74) Mandataire: Office Freylinger
(86) Numéro de dépôt international: PCT/EP2016/070379
(87) Numéro de publication internationale: WO 2017/037044

(56) Documents cités:
- EP-A1- 2 623 538
- US-A- 3 994 853
- US-A- 4 698 411
- US-A- 5 977 217
- US-A1- 2015 098 971

## Description

### Domaine technique

La présente invention concerne une composition cosmétique anhydre améliorée applicable sur les ongles ou les faux-ongles. Cette composition forme un film dur, brillant et tenace. Cette composition peut être colorée ou elle peut être transparente et s'appliquer sur les ongles ou sur un vernis coloré.

### Etat de la technique

Les différentes propriétés que l'on demande à un vernis à ongles sont les suivantes :
- Bonne application, avec un pinceau de taille et forme adéquate. Le pinceau permet d'obtenir une surface lisse, sans stries avec une épaisseur constante afin d'obtenir un film et une coloration homogènes.
- Un temps de séchage approprié.
- Un durcissement à coeur de l'ordre de quelques minutes au plus.
- Une tenue sur l'ongle dans le temps en retardant l'écaillement du vernis ou son usure en bout d'ongle.
- Une brillance importante à l'application, c'est à dire quelques minutes après le séchage définitif de la composition.
- Une tenue de la brillance dans le temps, c'est à dire une brillance diminuant le moins possible dans les jours qui suivent l'application.

Le paramètre d'application dépend plus particulièrement de la rhéologie du milieu mais aussi de la nature de la teinte. La viscosité de la formulation dépend en effet des différents ingrédients utilisés dans la formulation et de leur dosage au sein de cette formulation. L'application peut également dépendre des matières colorantes utilisées dans la formulation colorée mais aussi de la qualité de l'applicateur.

Le temps de séchage est principalement dépendant des solvants utilisés dans la formulation et en particulier leur température d'ébullition et leur tension de vapeur. L'utilisation de différents solvants ayant des propriétés physico-chimiques différentes permet de moduler le temps de séchage et de durcissement du film. La nature des autres ingrédients utilisés dans la formulation peut également influer sur la vitesse du temps de séchage et son durcissement à coeur.

La durabilité du vernis sur l'ongle ou tenue est principalement due aux agents non volatiles de la formulation tels que les résines, la nitrocellulose ou les différents additifs annexes.

La brillance est apportée par les différents constituants non volatils du vernis à ongles comme les résines ou la nitrocellulose. Toutefois, ce paramètre est fortement affecté par les composés solides non solubles compris dans la formulation. Parmi ces composés solides non solubles, on citera notamment les pigments qui entraînent par leur caractéristiques physico-chimiques et leur nature une diminution de la brillance par rapport à la même formulation non pigmentée. Les différentes étapes mises en oeuvre dans la préparation des pigments avant leur utilisation dans le vernis à ongles permettent plusieurs actions. Ces actions sur les pigments sont les suivantes :
- Le mouillage, qui permet de déplacer et d'éliminer l'air ou l'humidité qui se trouve à la surface du pigment.
- La dispersion qui permet de :
   - Casser les agglomérats et les agrégats de pigments ; les agglomérats correspondent à l'association de structures primaires de pigments par des contacts de surface, les agrégats correspondent plutôt à une structure secondaire ayant pour origine l'association des structures primaires par des contacts bord à bord
   - Distribuer de façon homogène les particules primaires des pigments afin d'éviter la reformation des structures secondaires
- La stabilisation de la dispersion qui évite le retour des agrégats.

D'une manière générale dans le domaine des vernis à ongles, il existe une demande constante de nouveaux produits ayant des propriétés améliorées, notamment au niveau de la facilité d'application, de la rapidité de séchage, de la durabilité, de l'esthétique, etc. De plus en plus, on observe aussi une sensibilité croissante des utilisateurs et utilisatrices pour des produits plus respectueux de l'environnement.

### Objet de l'invention

Un objet de la présente invention concerne par conséquent le développement de formulations de vernis à ongles colorées ou transparentes intégrant des composants disposant de propriétés adéquates et qui sont en outre plus respectueux de l'environnement.

### Description générale de l'invention

Selon un premier aspect de la présente invention, il est proposé d'utiliser un polyester copolymère d'acide citrique C(OH)(CO₂H)(CH₂CO₂H)₂, de propylène glycol CH(CH₃)(OH)-CH₂-OH, d'acide succinique HO₂C-(CH₂)₂-CO₂H et d'acide laurique CH₃-(CH₂)₁₀-CO₂H à titre de résine dans une composition cosmétique anhydre applicable sur les (faux) ongles, respectivement pour vernis à ongles ou de soins des ongles. Ledit polyester est donc un copolymère comprenant des monomères d'acide citrique, des monomères de propylène glycol, des monomères d'acide succinique et des monomères d'acide laurique, de préférence il est constitué uniquement de ces quatre types de monomères.

Un deuxième aspect de l'invention concerne des compositions cosmétiques anhydres applicables sur les (faux) ongles, respectivement pour vernis à ongles ou de soins des ongles, comprenant un tel polyester copolymère d'acide citrique, de propylène glycol, d'acide succinique et d'acide laurique. Ces compositions peuvent donc être des compositions prêtes à l'emploi ou des compositions servant de formulation de base à toute sorte de compositions prêtes à l'emploi.

En effet, il a été non seulement constaté qu'une telle résine offre les qualités attendues pour ce type d'ingrédient en apportant le collant nécessaire à la formulation, en permettant de réaliser une composition cosmétique brillante et en assurant une longue tenue sur l'ongle. Mais aussi que cette résine peut être préparée avec des monomères provenant entièrement de sources naturelles renouvelables. Cette résine peut ainsi être qualifiée de « bio » et permet d'obtenir le niveau de performances atteint par les résines issues de la pétrochimie.

Une résine préférée de ce type est par exemple celle commercialisée sous le nom de Natipol 1303 par l'entreprise Mäder. Cette résine est disponible notamment sous la forme d'une solution dans l'acétate de butyle, par exemple sous la forme d'un mélange résine/acétate de butyle : 9/1 ayant une viscosité de l'ordre de 10000 mPa.s à 20°C (méthode Noury). Cette résine est compatible avec la nitrocellulose et a une acidité résiduelle de 45-55 mg de KOH par gramme.

Il est important de noter que les éléments monomères mis en oeuvre pour la fabrication de la résine utile selon l'invention désignée copolymère acide citrique/propylène glycol/acide succinique/acide laurique peuvent être entièrement obtenus par l'extraction contrôlée de végétaux et s'inscrivent donc dans le cadre de ressources renouvelables, à l'opposé d'autres ingrédients connus issus de la pétrochimie.

D'une manière générale, les vernis à ongles comportent un certain nombre d'ingrédients ayant des propriétés et des fonctions définies. Parmi ces ingrédients, nous citerons les solvants, les filmogènes, les résines, les plastifiants, les agents de rhéologie, les agents de coloration ainsi que divers additifs.

D'une manière préférée, ladite résine copolymère est intégrée en une concentration allant de 0.2 à 15 % en poids, de préférence de 0.5 % à 10 % en poids par rapport à la composition cosmétique.

Dans une variante préférée, les compositions selon l'invention comprennent en outre un ou plusieurs solvants, un ou plusieurs plastifiants et/ou au moins un filmogène.

Dans une variante préférée, les compositions selon l'invention comprennent en outre au moins un agent de rhéologie et/ou au moins un agent de coloration.

Dans une variante préférée, les compositions selon l'invention comprennent en outre d'autres additifs comme les absorbants UV, les modificateurs de surface ou les additifs traitants de l'ongle.

Dans encore un aspect supplémentaire, l'invention concerne également un procédé de préparation d'une telle composition cosmétique anhydre, notamment pour vernis à ongles ou de soins des ongles, dans lequel on mélange la résine copolymère acide citrique/propylène glycol/acide succinique/acide laurique avec au moins un solvant organique cosmétiquement acceptable, de préférence avec au moins un agent filmogène, au moins un plastifiant, au moins un agent de rhéologie et au moins un agent de coloration dans le cas de la préparation d'une composition colorée. En outre, lors de la préparation de cette composition, un ou plusieurs additifs tels que décrits ci-avant pourront être ajoutés.

Les solvants généralement utilisés dans les vernis à ongles sont des solvants organiques volatiles ayant des tensions de vapeur élevées permettant un séchage par évaporation le plus rapide possible.

Les solvants utilisables dans le cadre de l'invention sont choisis par exemple parmi les suivants : l'acétate de butyle, l'acétate d'isobutyle, l'acétate de propyle, l'acétate de méthyle, l'acétate d'éthyle, le toluène, l'isopropanol, le diacétone alcool, l'éthanol dénaturé (avec la méthyléthylcétone ou le diéthyléther), le n-butanol, les hydrocarbures linéaires ou cycliques, comme l'hexane, le cyclohexane, l'heptane, l'isododécane. On citera également les dérivés de paraffine de type Isopar qui sont des dérivés paraffiniques qui ont des chaînes carbonées de taille variable, ainsi que les glycols comme l'éthylène glycol, le propylène glycol, le dipropylène glycol ou le tripropylène glycol.

Les solvants ou leurs mélanges peuvent être utilisés entre 5 et 80 % en poids de la formulation totale. Ils sont sélectionnés selon leurs propriétés physico-chimiques (température d'ébullition par exemple) et leur aptitude à solubiliser les autres constituants de la formulation. Ils seront également sélectionnés selon leurs propriétés organoleptiques (odeur principalement) et leurs possibles utilisations selon les réglementations en vigueur.

Les agents filmogènes sont sélectionnés pour leur aptitude à former un film poreux après séchage (évaporation partielle ou totale du ou des solvants). Les différents filmogènes utilisables dans l'invention sont les suivants :
- la nitrocellulose ayant un taux d'azote compris entre 11.8 et 12.3, il s'agit d'un grade de nitrocellulose soluble dans les solvants de type ester.
- La nitrocellulose ayant un taux d'azote inférieur 11.8, il s'agit d'un grade de nitrocellulose soluble dans les solvants de type alcool.

La nitrocellulose se présente sous la forme d'une poudre blanche humide. La nitrocellulose est en effet commercialisée sous forme « mouillée » (ou « damped ») avec plusieurs types de solvants comme par exemple l'éthanol ou l'isopropanol. La nitrocellulose mouillée à l'isopropanol sera préférée pour l'utilisation dans des compositions de vernis à ongles.

D'autres agents filmogènes utilisables selon l'invention sont :
- La cellulose acétate butyrate. Il existe de nombreux grades de cet agent filmogène qui se différencient par le taux d'estérification de la cellulose mais aussi par le rapport du nombre de fonction ester acétate et du nombre de fonction ester butyrate. Comme pour la nitrocellulose, la cellulose acétate butyrate peut également se différencier par le nombre d'unités cellulose du polymère.
- L'éthylcellulose de différents grades de viscosité.
- L'hydroxypropylcellulose de différents grades de viscosité.

Les agents filmogènes sont utilisés seuls ou sous la forme de mélange dans la composition dans des proportions allant de 5% à 25% en poids, de préférence 6 à 20 % en poids, en particulier de 7,5 à 15 % en poids de la composition totale.

Comme les résines copolymères utiles selon l'invention, les résines connues permettent de donner du collant et de l'adhérence au film sur l'ongle. Si nécessaire ou souhaité, d'autres résines peuvent être utilisées dans les compositions selon l'invention en plus des résines copolymères utiles selon l'invention, notamment les suivantes :
- La résine tosylamide/formaldéhyde (résine toluènesulfonamide/formaldéhyde) vendue sous la dénomination commerciale Ketjenflex MH, Ketjenflex MS-80 par Akzo Nobel ou encore Sulfonex par Estron.
- La résine tosylamide/époxy (résine toluènesulfonamide/époxy) vendue sous la dénomination commerciale de Lustrabrite S ou Lustrabrite S-70 ou Nagellite 3050 par Telechemische ou encore sous la dénomination polytex resin par Estron.
- Copolymère acide adipique/néopentyl glycol/anhydride trimellitique vendu sous la dénomination commerciale Uniplex 670-P par Unitex ou encore Chemol 509-9514 par Cook Composites
- Copolymère d'anhydride phtalique/glycérine/glycidyl décanoate
- Copolymère d'anhydride phtalique/anhydride trimellitique/glycols vendu sous la dénomination commerciale Polynex Resin par Estron ou encore 7809 Polynex Resin par Degen
- Copolymère de glycérine/acide phtalique
- Copolymère styrène/acrylate/acrylonitrile
- Polymères et copolymères d'acrylates
- Copolymères d'acrylates et de styrène
- Sucrose acétate isobutyrate vendu sous la dénomination commerciale SAIB par Eastman
- Résine polyvinylbutyral
- Résine Colophane appelée aussi Rosin obtenue par distillation de la résine de pin.

En plus de leur action collante, la plupart de ces résines ont également une action plastifiante. Ces résines peuvent être utilisées dans la formule (en plus de la ou des résines copolymères de l'invention) seules ou sous la forme de mélanges dans des quantités allant de 0.2 à 15% en poids, de préférence 0.25 à 12 % en poids, en particulier de 0.5 à 10 % en poids de la composition totale.

Les plastifiants sont des composés ayant une température d'ébullition élevée qui ne s'évaporent donc pas lors du séchage du vernis à ongles. Ils sont utilisés pour moduler la dureté du film formé afin d'obtenir les caractéristiques physico-chimiques du film souhaitées et représentent en général de 2 à 10 % en poids, de préférence 2,5 à 7,5 % en poids, en particulier de 3 à 6 % en poids de la composition totale.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate, etc.

Les compositions colorées selon l'invention comprendront un ou plusieurs agents de rhéologie. Parmi ces agents de rhéologie, on citera notamment les dérivés d'argile modifiés comme le stéaralkonium hectorite ou le stéaralkonium bentonite. Les oxydes de silice appelées également silices pyrogénées peuvent également être utilisées comme agent de rhéologie dans les compositions selon l'invention. Ces additifs de rhéologie seront de préférence utilisés seuls ou en combinaison de 0.1 à 3 % en poids total de la formulation. Ces additifs permettent en particulier la suspension des particules insolubles dans le vernis tels que les pigments ou encore les nacres.

La composition selon l'invention pourra comprendre en outre de 0.1 à 10 % en poids, de préférence 0.2 à 8 % en poids, en particulier de 0.25 à 7,5 % en poids de la composition totale d'une ou de plusieurs matières colorantes ou agents de coloration, comme les pigments et les particules nacrantes (nacres), les glitters ou les particules métalliques d'aluminium ou autres.

Parmi les pigments utilisables dans la composition selon l'invention on citera le dioxyde de titane (CI 77891), l'oxyde de fer noir (CI 77499), l'oxyde de fer rouge (CI 77491 ), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention. Les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaques de tailles variées. Parmi les nacres utilisables dans l'invention, on peut citer :
- les nacres préparées à partir de mica naturel et ayant subi des opérations de coating successives,
- les nacres préparées à partir de mica synthétique appelé également synthetic fluorphlogopite, également formées de couches successives d'oxydes métalliques,
- les nacres préparées à partir de calcium sodium borosilicate,
- les nacres préparées à partir d'oxyde de silice.

Les glitters utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Les compositions selon l'invention pourront également comprendre divers additifs comme les absorbants UV, les modificateurs de surface ou encore les actifs traitants de l'ongle.

Parmi les absorbants UV, on citera les absorbants de lumière UV qui permettent de protéger la formulation ou les agents colorants utilisés dans l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller de 0.1 à 1% en poids.

Parmi les additifs de modification de surface utilisables selon l'invention, on citera par exemple les cyclométhicones ou cyclopentasiloxane, les diméthicones ou le triméthylsiloxysilicate.

Ces additifs de surface sont généralement utilisés dans des faibles quantités dans la formulation allant de 0.01 à 0.5 % en poids total de la formulation.

On citera également les additifs dits « traitants » de l'ongle utilisables dans la composition selon l'invention. Les additifs traitants peuvent représenter de 0.0001% à 5%, de préférence de 0.01% à 1% en poids de la composition totale. Parmi ces additifs « traitants », on citera de manière non exhaustive :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane vendu sous la dénomination commerciale Pro-DSB par Exsymol,
- la myrrhe,
- les acides aminés soufrés comme la cystéine, ses sels et leurs dérivés, la cystine, le glutathion. Les acides aminés soufrés sont en effet capables de créer des cross-linkings entre la kératine et les groupes SH libres de ces dérivés soufrés,
- la kératine hydrolysée d'origine végétale comme le Prosina de Croda,
- les alpha-hydroxyacides comme l'acide citrique, l'acide ascorbique,
- la biotine,
- l'urée et la diméthylurée.

Les compositions cosmétiques selon l'invention sont des compositions cosmétiques anhydres et ne contiennent pas d'eau délibérément ajoutée comme solvant. Il est cependant possible que de faibles quantités d'eau soient contenues dans l'un ou l'autre des ingrédients. Dans le contexte de la présente invention, des compositions cosmétiques comprenant de tels ingrédients seront néanmoins qualifiées d'anhydres.

### Exemples

Le Tableau 1 ci-dessous décrit un exemple de formulation colorée utilisant la nouvelle résine selon l'invention.

**Tableau 1**

| | |
|---|---|
| Acétate d'éthyle | 29,10% |
| Acétate de butyle | 30,50% |
| Nitrocellulose (70%) | 16,00% |
| Résine selon l'invention | 10,00% |
| Acétyl tributyl citrate | 6,50% |
| Stearalkonium hectorite | 0,60% |
| Etocrylène | 0,30% |
| DC Red 6 | 2,00% |
| DC Red 34 | 2,00% |
| Dioxyde de titane | 3,00% |
| Total | 100,00% |

Le tableau 2 ci-dessous décrit un exemple de formulation incolore et transparente

**Tableau 2**

| | |
|---|---|
| Acétate d'éthyle | 48,40% |
| Acétate de butyle | 13,80% |
| Nitrocellulose (70%) | 18,00% |
| Résine selon l'invention | 10,00% |
| Acétyl tributyl citrate | 6,50% |
| Copolymère styrène/acrylique | 3,00% |
| Etocrylène | 0,30% |
| Total | 100,00% |

## Revendications

1. Utilisation d'un polyester qui est un copolymère d'acide citrique, de propylène glycol, d'acide succinique et d'acide laurique à titre de résine dans une composition cosmétique anhydre applicable sur les ongles.

2. Composition cosmétique anhydre pour vernis à ongles ou le soin des ongles comprenant une résine copolymère d'acide citrique, de propylène glycol, d'acide succinique et d'acide laurique.

3. Composition cosmétique selon la revendication 2, comprenant ladite résine copolymère en une concentration allant de 0.2 % à 15 % en poids, de préférence de 0.5 % à 10 % en poids.

4. Composition cosmétique selon la revendication 2 ou 3, comprenant en outre un ou plusieurs solvants cosmétiquement acceptables, un ou plusieurs plastifiants et au moins un agent filmogène.

5. Composition cosmétique selon l'une quelconque des revendications 2 à 4, comprenant en outre au moins un agent de rhéologie et/ou au moins un agent de coloration.

6. Composition cosmétique selon l'une quelconque des revendications 2 à 5, comprenant en outre d'autres additifs comme les absorbants UV, les modificateurs de surface ou les additifs traitants de l'ongle.

7. Procédé de préparation d'une composition cosmétique anhydre selon l'une quelconque des revendications 2 à 6, dans lequel on mélange ladite résine copolymère d'acide citrique, de propylène glycol, d'acide succinique et d'acide laurique à au moins un solvant organique cosmétiquement acceptable, ainsi que de préférence un ou plusieurs plastifiants et au moins un agent filmogène.

8. Procédé selon la revendication 7, dans lequel on ajoute en outre au moins un agent de rhéologie et/ou au moins un agent de coloration à la composition cosmétique.

9. Procédé selon la revendication 7 ou 8, dans lequel on ajoute en outre d'autres additifs comme les absorbants UV, les modificateurs de surface ou les additifs traitants de l'ongle.

## Patentansprüche

1. Verwendung eines Polyesters, der ein Copolymer von Citronensäure, Propylenglykol, Bernsteinsäure und Laurinsäure ist, als Harz in einer wasserfreien kosmetischen Zusammensetzung, die auf die Nägel anwendbar ist.

2. Wasserfreie kosmetische Zusammensetzung als Nagellack oder zur Nagelpflege, die ein Copolymerharz von Citronensäure, Propylenglykol, Bernsteinsäure und Laurinsäure umfasst.

3. Kosmetische Zusammensetzung nach Anspruch 2, die das Copolymerharz in einer Konzentration umfasst, die von 0,2 Gew.-% bis 15 Gew.-%, vorzugsweise von 0,5 Gew.-% bis 10 Gew.-% reicht.

4. Kosmetische Zusammensetzung nach Anspruch 2 oder 3, die außerdem ein oder mehrere kosmetisch verträgliche Lösungsmittel, einen oder mehrere Weichmacher und mindestens einen Filmbildner umfasst.

5. Kosmetische Zusammensetzung nach einem der Ansprüche 2 bis 4, die außerdem mindestens ein Rheologiemittel und/oder mindestens ein Farbmittel umfasst.

6. Kosmetische Zusammensetzung nach einem der Ansprüche 2 bis 5, die außerdem weitere Additive umfasst, wie UV-Absorber, Oberflächenmodifikatoren oder Nagelbehandlungsadditive.

7. Verfahren zur Herstellung einer wasserfreien kosmetischen Zusammensetzung nach einem der Ansprüche 2 bis 6, wobei das Copolymerharz von Citronensäure, Propylenglykol, Bernsteinsäure und Laurinsäure mit mindestens einem kosmetisch verträglichen organischen Lösungsmittel sowie vorzugsweise einem oder mehreren Weichmachern und mindestens einem Filmbildner gemischt wird.

8. Verfahren nach Anspruch 7, wobei außerdem mindestens ein Rheologiemittel und/oder mindestens ein Farbmittel der kosmetischen Zusammensetzung zugegeben werden.

9. Verfahren nach Anspruch 7 oder 8, wobei außerdem weitere Additive zugegeben werden, wie UV-Absorber, Oberflächenmodifikatoren oder Nagelbehandlungsadditive.

## Claims

1. Use of a polyester which is a copolymer of citric acid, propylene glycol, succinic acid and lauric acid as resin in an anhydrous cosmetic composition applicable to the nails.

2. Anhydrous cosmetic composition for nail varnish or nail care comprising a copolymer resin of citric acid, propylene glycol, succinic acid and lauric acid.

3. Cosmetic composition according to claim 2, comprising said copolymer resin in a concentration of from 0.2% to 15% by weight, preferably from 0.5% to 10% by weight.

4. Cosmetic composition according to claim 2 or 3, furthermore comprising one or more cosmetically acceptable solvents, one or more plasticisers and at least one film-forming agent.

5. Cosmetic composition according to any one of claims 2 to 4, furthermore comprising at least one rheological agent and/or at least one colouring agent.

6. Cosmetic composition according to any one of claims 2 to 5, furthermore comprising other additives such as UV absorbers, surface modifiers or nail treatment additives.

7. Method for preparing an anhydrous cosmetic composition according to any one of claims 2 to 6, in which said copolymer resin of citric acid, propylene glycol, succinic acid and lauric acid is mixed with at least one cosmetically acceptable organic solvent, preferably together with one or more plasticisers and at least one film-forming agent.

8. Method according to claim 7, in which at least one rheological agent and/or at least one colouring agent is furthermore added to the cosmetic composition.

9. Method according to claim 7 or 8, in which other additives such as UV absorbers, surface modifiers or nail treatment additives are furthermore added.
